Europäisches Patentamt

European Patent Office

Office européen des brevets

(19)

(11) EP 1 230 893 A2

(12) **EUROPÄISCHE PATENTANMELDUNG**

(43) Veröffentlichungstag:
14.08.2002 Patentblatt 2002/33

(51) Int Cl.$^7$: **A61B 5/0456**

(21) Anmeldenummer: 01130148.8

(22) Anmeldetag: 19.12.2001

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **07.02.2001 DE 10105431**

(71) Anmelder: **BIOTRONIK Mess- und Therapiegeräte GmbH & Co Ingenieurbüro Berlin 12359 Berlin (DE)**

(72) Erfinder:
- **Köhler, Bert-Uwe, Dr.**
  **14612 Berlin-Falkensee (DE)**
- **Orglmeister, Reinhold, Prof. Dr.-Ing.**
  **13467 Berlin (DE)**

(74) Vertreter: **Hübner, Gerd, Dipl.-Phys. et al Rau, Schneck & Hübner Patentanwälte Königstrasse 2 90402 Nürnberg (DE)**

(54) **Signalauswerteverfahren zur Detektion von QRS-Komplexen in Elektrokardiogramm-Signalen**

(57)    Ein Signalauswerteverfahren zur Detektion von QRS-Komplexen in Elektrokardiogramm-Signalen weist folgende Verfahrensschritte auf:

- Abtasten des EKG-Signals (4) und Umwandlung in diskrete, zeitlich aufeinanderfolgende Signalwerte ($x(n)$, $x_{fq}(n)$),
- Vergleichen der Signalwerte ($x(n)$, $x_{fq}(n)$) mit einer adaptiv aus diesen ermittelten Schwellenwertfunktion ($K(n)$),

- Feststellen einer Häufigkeitszahl ($D(n)$) innerhalb eines definierten Segmentes der aufeinanderfolgenden Signalwerte ($x(n)$, $x_{fq}(n)$), mit der vorzugsweise die Beträge der Signalwerte ($x(n)$, $x_{fq}(n)$) die Schwellenwertfunktion ($K(n)$) unterschreiten, und
- Vergleichen der ermittelten Häufigkeitszahl ($D(n)$) mit einem definierten Grenzwert ($\Theta$), wobei ein Unterschreiten des Grenzwerts ($\Theta$) signifikant für das Vorliegen eines QRS-Komplexes (5, 6, 7) in dem definierten Segment des EKG-Signals (4) ist.

FIG. 2

**Beschreibung**

Die Erfindung betrifft ein Signalauswerteverfahren zur Detektion von QRS-Komplexen in Elektrokardiogramm-(EKG- und IEGM-)Signalen.

Zum Hintergrund der Erfindung ist festzuhalten, daß die automatische Analyse von EKG-Signalen zur Perfektionierung der Funktionsfähigkeit von Herzschrittmachern und Defibrillatoren eine immer größere Rolle spielt. Solche kardiologischen Geräteimplantate jüngerer Bauart bieten dementsprechend auch die Fähigkeit einer EKG-Analyse. In diesem Zusammenhang hat die Erkennung von QRS-Komplexen und R-Zacken in EKG-Signalen einen überragenden Stellenwert. Diese Bedeutung resultiert aus der vielfältigen Verwendbarkeit der Information über das zeitliche Auftreten des QRS-Komplexes, z. B. bei der Untersuchung der Herzratenvariabilität, bei der Klassifikation und bei der Datenkompression sowie als Basissignal für sekundäre Anwendungen. Überhaupt nicht oder falsch detektierte QRS-Komplexe und R-Zacken sind dabei problematisch im Hinblick auf die Leistungsfähigkeit der auf die Detektion folgenden Verarbeitungs- und Analysestufen.

Einen breiten Überblick über bekannte Signalauswerteverfahren zur Detektion von QRS-Komplexen in EKG-Signalen bietet der Fachaufsatz von Friesen et al. "A Comparison of the Noise Sensitivity on Nine QRS Detection Algorithms" in IEEE Transaction on Biomedical Engineering, Vol. 37, Nr. 1, Januar 1990, Seiten 85 - 98. Die dort vorgestellten Signalauswerte-Algorithmen basieren durchgehend auf einer Auswertung der Amplitude, der ersten Ableitung des Signals sowie dessen zweiter Ableitung. Bei den vorgestellten Algorithmen wird unterschieden zwischen solchen, die eine Analyse der Amplitude und der ersten Ableitung vornehmen, solchen, die nur die erste Ableitung bewerten und solchen, die die erste und zweite Ableitung in Betracht ziehen. Kurz umrissen wird bei allen Algorithmen abgeprüft, ob der jeweilige Signalparameter vorgegebene Schwellen über- oder unterschreitet, wonach bei Auftreten eines solchen Ereignisses mit einem vorgegebenen Schema das Auftreten weiterer definierter Ereignisse abgeprüft und bei Erfüllung bestimmter Kriterien auf das Vorliegen eines QRS-Komplexes geschlossen wird.

Ein anderer Aspekt bei der Signalauswertung zur Detektion von QRS-Komplexen ist bei der Implementierung solcher Verfahren in implantierten Herzschrittmachern zu beachten. Im Hinblick auf die naturgemäß bei solchen Geräten gegebenen Beschränkungen bei der Energieversorgung und der Rechenkapazität ist darauf zu achten, daß zur Detektion von QRS-Komplexen möglichst einfache Algorithmen mit möglichst wenigen Rechenoperationen auf der Basis von ganzen Zahlen anstatt von reellen Zahlen durchzuführen sind.

Ferner wurden zur QRS-Erkennung Signalverarbeitungsmethoden aus den Gebieten der linearen und nichtlinearen Filterung, der Wavelet-Transformation, der künstlichen neuronalen Netze und der genetischen Algorithmen angewendet. Bei hohen Signal-Stör-Abständen und nicht pathologischen Signalen, also im Falle guter Signalverhältnisse, erzielten diese Auswerteverfahren zuverlässige Ergebnisse. Waren keine solchen Verhältnisse gegeben, konnte die Leistungsfähigkeit der Auswerteverfahren unter Umständen drastisch absinken, was im Hinblick auf eine zuverlässige Funktionsweise eines Schrittmachers natürlich nicht akzeptabel ist.

Schließlich ist aus der älteren, jedoch nachveröffentlichten deutschen Patentanmeldung 100 11 733.3 der Anmelderin eine QRS-Erkennung auf der Basis von Nulldurchgangszählungen bekannt. Dabei werden folgende Verfahrensschritte durchgeführt:

- Abtasten des Signals und Umwandlung in diskrete, zeitlich aufeinanderfolgende Signalwerte,
- Hochpaßfilterung der abgetasteten Signalwerte,
- Bestimmung des Vorzeichens jedes Signalwerts,
- laufendes Abprüfen der Vorzeichen aufeinanderfolgender Signalwerte auf Vorliegen eines Nulldurchgangs zwischen zwei aufeinanderfolgenden Signalwerten,
- Ermittlung der Anzahl der Nulldurchgänge in einem definierten Segment der aufeinanderfolgenden Signalwerte, und
- Vergleichen der ermittelten Nulldurchgangszahl mit einem definierten Schwellenwert, wobei ein Unterschreiten des Schwellenwertes signifikant für das Vorliegen eines QRS-Komplexes in dem definierten Segment des Signalverlaufes ist.

Um dabei im Bereich außerhalb der QRS-Komplexe eine signifikant hohe Nulldurchgangszahl zu erhalten, wird dort zu dem Hoch- oder Bandpaß-gefilterten und quadrierten EKG-Signal ein hochfrequentes Überlagerungssignal mit im Vergleich zur Amplitude des QRS-Komplexes niedriger Amplitude addiert.

Diese Vorgehensweise steht der eingangs erläuterten Forderung nach möglichst einfachen Algorithmen natürlich entgegen.

Ausgehend von den geschilderten Problematiken liegt der Erfindung die Aufgabe zugrunde, ein Signalauswerteverfahren zur Detektion von QRS-Komplexen in EKG-Signalen anzugeben, das mit vergleichsweise geringer Rechenkapazität und auch bei problematischen Signalverhältnissen einsetzbar ist, dabei jedoch zuverlässige Detektionsergebnisse liefert.

Diese Aufgabe wird durch die im Patentanspruch 1 angegebenen kennzeichnenden Verfahrensschritte wie folgt gelöst:

- Abtasten des EKG-Signals und Umwandlung in diskrete, zeitlich aufeinanderfolgende Signalwerte,
- Vergleichen der Signalwerte mit einer adaptiv aus diesen ermittelten Schwellenwertfunktion,
- Feststellen einer Häufigkeitszahl innerhalb eines definierten Segmentes der aufeinanderfolgenden Signalwerte, mit der vorzugsweise die Beträge der Signalwerte die Schwellenwertfunktion unterschreiten, und
- Vergleichen der ermittelten Häufigkeitszahl mit einem definierten Grenzwert, wobei ein Unterschreiten des Grenzwerts signifikant für das Vorliegen eines QRS-Komplexes in dem definierten Segment des EKG-Signals ist.

Kernpunkt des erfindungsgemäßen Verfahrens ist die Anwendung eines Schwellenwertvergleichs und einer nachfolgenden Häufigkeitszählung, wobei die Morphologie des QRS-Komplexes ausgenutzt wird. Der QRS-Komplex im EKG-Signal ist nämlich durch eine relativ hochamplitudige Oszillation charakterisiert, die den Signalverlauf ausgeprägt von der in der Regel verrauschten und mit einem Offset beaufschlagten Basis-Linie des Elektrokardiogramms wegführt. Die Frequenz dieser kurzen Oszillation liegt in einem Bereich, in dem andere Signalkomponenten, wie die P- und T-Welle, nur geringen Einfluß ausüben und vorzugsweise durch Vorfilterung - z. B. eine Hoch- oder Bandpaßfilterung - entfernt werden können. Nach der Unterdrückung dieser tieffrequenten Signalkomponenten ergeben sich aufgrund von höherfrequentem Rauschen Signalschwankungen um die Basis-Linie, die in dem Bereich, wo kein QRS-Komplex auftritt, das EKG-Signal dominieren. Der QRS-Komplex erscheint dann in diesem Signalkontext als langsame, nur kurz andauernde Schwingung hoher Amplitude, die von der Basis-Linie des EKG-Signals signifikant wegführt. Werden also die Signalwerte mit einer das Signalrauschen repräsentierenden Schwellenwertfunktion verglichen, so sind die Beträge der Signalwerte außerhalb des QRS-Komplexes meist kleiner als diese Schwellenwertfunktion. Insoweit ist die Häufigkeitszahl, mit der die Beträge der Signalwerte die Schwellenwertfunktion unterschreiten, groß. Im Bereich des QRS-Komplexes überschreiten nun die Beträge der Signalwerte die Schwellenwertfunktion, und zwar signifikant. Es stellt sich also eine geringe Häufigkeitszahl für Unterschreitungen der Schwelle im Zuge des QRS-Komplexes ein. Durch ein Vergleichen der ermittelten Häufigkeitszahl mit einem definierten Grenzwert kann also der QRS-Komplex selektiert werden. Ein Unterschreiten des Grenzwertes signalisiert nämlich das für den QRS-Komplex typische Überschreiten der Schwellenwertfunktion.

Das erfindungsgemäße Verfahren der QRS-Komplex-Erkennung hat sich als robust gegenüber Rauschstörungen und rechentechnisch besonders einfach realisierbar erwiesen. Insoweit eignet es sich besonders für die Implementierung bei der Realzeit-Analyse von EKG-Signal-Morphologien in Herzschrittmachern.

Die bereits erwähnte Hochpaßfilterung wird vorzugsweise mit einer unteren Durchlaßfrequenz von 18 Hz vorgenommen. Damit können die tieffrequenten Komponenten, wie die P- und T-Welle sowie eine Grundliniendrift unterdrückt werden. Ferner wird der QRS-Komplex dadurch zur Signalkomponente mit der niedrigsten Frequenz, die das Signal während ihres Auftretens dominiert.

Zur Vergrößerung des Signal-Stör-Abstandes kann ferner vorgesehen sein, die Signalwerte vor dem Vergleichen mit der Schwellenwertfunktion und der Häufigkeitszahl zu quadrieren. Damit werden kleine Signalbeträge relativ zu größeren Signalbeträgen abgeschwächt, was die Detektierbarkeit des QRS-Komplexes weiter verbessert.

Der Wert der Schwellenwertfunktion wird vorzugsweise adaptiv aus einer fließenden Mittelung der Bandpaßgefilterten und quadrierten Signalbeträge bestimmt.

Im folgenden wird das erfindungsgemäße Verfahren anhand der beigefügten Zeichnungen in einem Ausführungsbeispiel näher erläutert. Es zeigen:

Fig. 1    eine höchst schematische Darstellung des Signalverlaufs eines QRS-Komplexes in einem EKG-Signal, und

Fig. 2    ein Strukturschema des erfindungsgemäßen Signalauswerteverfahrens zur Detektion von QRS-Komplexen in EKG-Signalen.

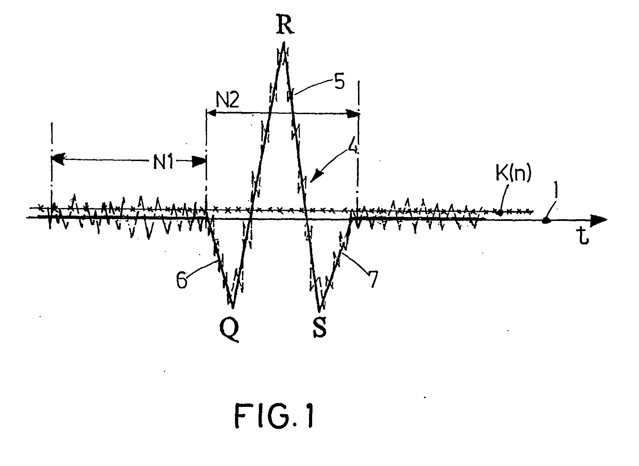

Wie aus Fig. 1 deutlich wird, besteht ein schematisch dargestellter QRS-Komplex aus einer relativ hochamplitudigen Oszillation um die Null-Linie 1, die das EKG-Signal 4 in der Q-Zacke 6 zuerst in negativer Richtung über die Null-Linie 1 wegführt. Anschließend wird das EKG-Signal 4 in der R-Zacke 5 mit einem steilen Anstieg in den positiven Bereich und einem anschließenden steilen Abfall wiederum in den negativen Bereich unter Ausbildung der S-Zacke 7 übergeführt.

Real ist das EKG-Signal 4 mit einem bestimmten Pegel verrauscht, was in Fig. 1 durch den strichlierten Signalverlauf angedeutet ist. Wird nun dieses verrauschte Signal 4 abgetastet und in diskrete, zeitlich aufeinanderfolgende Signalwerte umgewandelt und Bandpaß-gefiltert, so können diese Signalwerte mit der in Fig. 1 schematisch als mit Kreuzchen unterlegte Linie dargestellten Schwellenwertfunktion K(n) verglichen werden. Wie sich nun aus Fig. 1 anschaulich und modellhaft ableiten läßt, liegt im Bereich außerhalb des QRS-Komplexes der Betrag des EKG-Signals meist unterhalb dieser Schwellenwertfunktion K(n). Es ergeben sich also beispielsweise im Bereich N1 signifikant hohe Häufigkeiten für Signalbeträge |x(n)| unterhalb der Schwellenwertfunktion K(n).

Im Bereich der Q-Zacke 6 weicht nun der Betrag des EKG-Signals sehr stark von der Schwellenwertfunktion K(n) in positive Richtung ab. Die Häufigkeitszahl D(n) innerhalb des Segmentes N2 des QRS-Komplexes 6 für dieses Ereignis ist also wesentlich geringer als die Häufigkeitszahl innerhalb des Segments N1. Insoweit kann die Häufigkeitszahl D(n) für die Erkennung des QRS-Komplexes herangezogen werden. Dieser wird dann als vorliegend erkannt, wenn die Häufigkeitsanzahl D(n) einen definierten Grenzwert Θ unterschreitet.

Als ein Kernpunkt der Erfindung gegenüber dem Stand der Technik ist an dieser Stelle hervorzuheben, daß aufgrund der Feststellung der erörterten Häufigkeitszahl und deren Vergleich mit einem definierten Grenzwert nicht - wie im Stand der Technik - die Amplitude des EKG-Signals daraufhin überprüft wird, ob ein bestimmter Schwellenwert absolut überschritten wird, um daraus auf den QRS-Komplex zu schließen. Vielmehr wird gewissermaßen überprüft, wie lange das EKG-Signal eindeutig auf einer für das Vorliegen eines QRS-Komplexes sprechenden Seite einer Schwellenwertfunktion "verharrt". Erst das Vorliegen einer bestimmten Zeitdauer dieses Zustandes wird zum Rückschluß auf das Vorliegen eines QRS-Komplexes herangezogen. Demzufolge werden nur kurze, starke Meßschwankungen nicht als (falsche) QRS-Komplexe (sogenannte "False positive"-Fehler) erkannt.

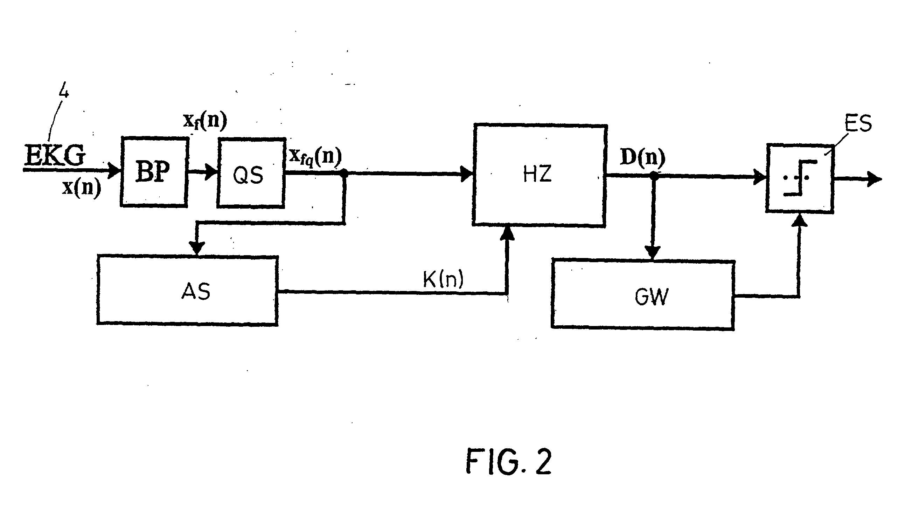

Der detaillierte Ablauf des erfindungsgemäßen Auswerteverfahrens wird nun im folgenden anhand der Fig. 2 näher erläutert.

Das EKG-Signal 4 wird abgetastet und in diskrete, zeitlich aufeinanderfolgende Signalwerte x(n) umgewandelt. Die Abtastrate beträgt beispielsweise $f_t$ = 360 Hz, d. h., daß das EKG-Signal in eine Folge von 360 Meßwerten pro Sekunde umgewandelt wird. Das abgetastete EKG-Signal x(n) wird dann eingangsseitig einer Bandpaßfilterung BP unterzogen, die der Entfernung aller Signalkomponenten, die nicht zum QRS-Komplex gehören, dient. Dazu zählen die P- und T-Welle sowie hochfrequentere Störungen, die z. B. von der bioelektrischen Muskelaktivität ausgehen. Der eingesetzte Filter BP ist linearphasig, nicht rekursiv und besitzt eine Bandpaßcharakteristik mit den Durchlaßfrequenzen $f_{g1}$ = 18 Hz und $f_{g2}$ = 27 Hz sowie den Sperrgrenzfrequenzen $f_{s1}$ = 2 Hz und $f_{s2}$ = 50 Hz. Die Filterordnung ist FO = 26. Die Gruppenlaufzeit des Bandpaßfilters BP entspricht dementsprechend 13 Abtastwerten und muß bei der Bestimmung des Zeitpunktes des QRS-Komplexes berücksichtigt werden.

Die so erhaltenen Signalwerte $x_f$(n) werden anschließend in einer Quadrierstufe QS gemäß folgender Beziehung quadriert:

$$| \, x_f(n) \, |^2$$

Die so aus den ursprünglichen Signalwerten x(n) durch eine Art Betragsbildung aufbereiteten Werte $x_{fq}$(n) werden nun einem Vergleicherkomplex HZ zugeführt, der diese Signalwerte mit einer adaptiv aus diesen ermittelten Schwellenwertfunktion K(n) vergleicht. In Fig. 2 ist der die Ermittlung der Schwellenwertfunktion K(n) betreffende Verfahrenskomplex mit AS gekennzeichnet. Darin wird ein geeigneter Wert für die Funktionskoeffizienten K(n) adaptiv aus den Signalwerten $x_{fq}$(n) geschätzt. Dazu werden fließend die Bandpaß-gefilterten und quadrierten Signalwerte mit Hilfe eines Gedächtnisfaktors
$\lambda_k$ (0 < $\lambda_k$ < 1) rekursiv gemittelt.

$$K(n) = \lambda_k \, K(n-1) + (1 - \lambda_k) \, x_{fq}(n) \cdot c$$

wobei c ein Konstante ist.

Empirisch ergeben sich als geeignete Werte $\lambda_k$ = 0,98 und c = 8.

Die durch den Gedächtnisfaktor $\lambda_k$ vorgegebene Mittelungszeit bestimmt im wesentlichen die Adaptionsgeschwindigkeit dieser Schätzung, wobei sowohl zu kurze als auch zu lange Mittelungssegmente die Leistungsfähigkeit des Signalauswerteverfahrens beeinträchtigen können.

Im Verfahrenskomplex HZ werden nun die Signalwerte $x_{fq}$(n) mit der Schwellenwertfunktion K(n) - wie angesprochen - verglichen. Dabei wird festgestellt, in welcher Richtung die Signalwerte $x_{fq}$(n) von der Schwellenwertfunktion K(n) abweichen. Daraus wird eine Häufigkeitsanzahl D(n) innerhalb dieses definierten Segmentes N festgestellt, die die Anzahl oder Häufigkeit von Ereignissen repräsentiert, bei denen die Beträge der Signalwerte $x_{fq}$(n) unterhalb der Schwellenwertfunktion liegen. D(n) kann auch auf rechengünstige Weise rekursiv über

$$D(n) = \lambda_D\, D(n-1) + (1 - \lambda_D)\, d(n) \text{ mit } d(n) = \begin{cases} 0 \text{ für } x_{fq}(n) \geq k(n) \\[2ex] 1 \text{ für } x_{fq}(n) < k(n) \end{cases}$$

ermittelt werden. Ein kleiner Wert für die Häufigkeitszahl D(n) spricht dafür, daß der Betrag des EKG-Signals 4 dauerhaft oberhalb der Schwellenwertfunktion K(n) liegt, was einen verläßlichen Parameter für das Vorliegen des QRS-Komplexes darstellt.

Im Zuge des erfindungsgemäßen Verfahrens muß also noch ein Grenzwert Θ ermittelt werden, dessen Unterschreiten signifikant für das Vorliegen eines QRS-Komplexes in dem definierten Segment des EKG-Signals 4 spricht. Θ(n) wird rekursiv aus D(n) berechnet mit

$$\Theta(n) = \lambda_\theta\, \Theta(n-1) + (1 - \lambda_\theta)\, D(n)$$

wobei ein Gedächtnisfaktor $0 < \lambda_\theta < 1$ verwendet wird. Dieser Gedächtnisfaktor kann beispielsweise zu $\lambda_\theta = 0{,}99$ gewählt werden. Liegt D(n) unterhalb des Grenzwertes Θ, ist ein QRS-Komplex erfaßt worden, andernfalls nicht.

Die Abprüfung, ob die vorstehende Bedingung erfüllt worden ist, findet in der Entscheiderstufe ES gemäß Fig. 2 statt.

Das erfindungsgemäße Auswerteverfahren kann durch Implementierung softwaremäßig in Form eines entsprechenden Auswerteprogramms, aber auch über eine hardwaremäßige Realisierung durch eine entsprechende elektronische Auswerteschaltung umgesetzt werden.


**Patentansprüche**

1.  Signalauswerteverfahren zur Detektion von QRS-Komplexen in Elektrokardiogramm-(EKG-)Signalen, **gekennzeichnet durch** folgende Verfahrensschritte:

    -   Abtasten des EKG-Signals (4) und Umwandlung in diskrete, zeitlich aufeinanderfolgende Signalwerte (x(n)),
    -   Vergleichen der Signalwerte ($x_f(n)$, $x_{fq}(n)$) mit einer adaptiv aus diesen ermittelten Schwellenwertfunktion (K(n)),
    -   Feststellen einer Häufigkeitszahl (D(n)) innerhalb eines definierten Segmentes der aufeinanderfolgenden Signalwerte ($x_f(n)$, $x_{fq}(n)$), mit der die Signalwerte ($x_f(n)$, $x_{fq}(n)$) die Schwellenwertfunktion (K(n)) unterschreiten, und
    -   Vergleichen der ermittelten Häufigkeitszahl (D(n)) mit einem definierten Grenzwert (Θ), wobei ein Unterschreiten des Grenzwertes (Θ) signifikant für das Vorliegen eines QRS-Komplexes (5, 6, 7) in dem definierten Segment des EKG-Signals (4) ist.

2.  Signalauswerteverfahren nach Anspruch 1, **dadurch gekennzeichnet, daß** die EKG-Signalwerte (x(n)) nach dem Abtasten einer Hochpaßfilterung, vorzugsweise einer Bandpaßfilterung (BP) unterworfen werden.

3.  Signalauswerteverfahren nach Anspruch 2, **dadurch gekennzeichnet, daß** die untere und obere Durchlaßgrenzfrequenz ($f_{g1}$, $f_{g2}$) des Bandpaßfilters (BP) bei ca. 18 Hz und ca. 27 Hz liegen.

4.  Signalauswerteverfahren nach einem der Ansprüche 1 bis 3, **dadurch gekennzeichnet, daß** die Signalwerte (x(n)) einer Betragsbildung unterworfen werden.

5.  Signalauswerteverfahren nach Anspruch 4, **dadurch gekennzeichnet, daß** die Signalwerte ($x_f(n)$) vor dem Vergleichen mit der Schwellenwertfunktion (K(n)) und Ermitteln der Häufigkeitszahl (D(n)) zur Betragsbildung quadriert werden.

6.  Signalauswerteverfahren nach einem der Ansprüche 1 bis 5, **dadurch gekennzeichnet, daß** der Wert der Schwellenwertfunktion (K(n)) adaptiv aus einer fließenden Mittelung der Signalwerte (x(n)), vorzugsweise der Bandpaßgefilterten und quadrierten Signalwerte ($x_{fq}(n)$), über eine durch den Gedächtnisfaktor ($\lambda_k$) vorgegebene Mitte-

lungsperiode (P) bestimmt wird.

7. Signalauswerteverfahren nach einem der Ansprüche 1 bis 6, **dadurch gekennzeichnet, daß** der für einen QRS-Komplex (5, 6, 7) signifikante Grenzwert ($\Theta$) der Häufigkeitszahl (D(n)) als adaptive Schwelle aus der Häufigkeitszahl (D(n)) selbst variabel eingestellt wird.

FIG.1

FIG. 2

EP 1 230 893 A2